(19) Europäisches Patentamt
European Patent Office
Office européen des brevets

(11) Veröffentlichungsnummer: 0 002 812
B1

(12) EUROPÄISCHE PATENTSCHRIFT

(45) Veröffentlichungstag der Patentschrift: 03.02.82

(21) Anmeldenummer: 78101806.4

(22) Anmeldetag: 21.12.78

(51) Int. Cl.³: C 07 D 263/14, C 07 D 413/12, A 01 N 43/76

# (54) Phenoxy-alkyl-oxazoline, ihre Herstellung, sie enthaltende Mittel sowie deren Verwendung als Herbizide.

(30) Priorität: 02.01.78 CH 2/78

(43) Veröffentlichungstag der Anmeldung: 11.07.79 Patentblatt 79/14

(45) Bekanntmachung des Hinweises auf die Patenterteilung: 03.02.82 Patentblatt 82/5

(84) Benannte Vertragsstaaten: BE CH DE FR GB IT NL

(56) Entgegenhaltungen:
BE - A - 862 325
US - A - 3 778 445

(73) Patentinhaber: CIBA-GEIGY AG
Patentabteilung Postfach
CH-4002 Basel (CH)

(72) Erfinder: Föry, Werner, Dr.
Benkenstrasse 65
CH-4054 Basel (CH)
Erfinder: Rempfler, Hermann, Dr.
Allschwilerweg 67
CH-4102 Binningen (CH)
Erfinder: Pissiotas, Georg, Dr.
Breslauerstrasse 8
D-7850 Lörrach (DE)
Erfinder: Rohr, Otto, Dr.
Kilbertweg 19
CH-4106 Therwil (CH)
Erfinder: Böhner, Beat, Dr.
Hügelweg 3
CH-4102 Binningen (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 812 B1

Phenoxy-alkyl-oxazoline, ihre Herstellung, sie enthaltende Mittel sowie deren Verwendung als Herbizide

Die vorliegende Erfindung betrifft neue, herbizid wirksame, kernsubstituierte Phenoxy-alkyl-oxazoline, Verfahren zu ihrer Herstellung, ferner herbizide Mittel, di diese neuen Verbindungen als Wirkstoffe enthalten, sowie Verfahren zur selektiven Bekämpfung grasartiger Unkräuter in Kulturpflanzungen.

Die neuen Wirkstoffe entsprechen der Formel I

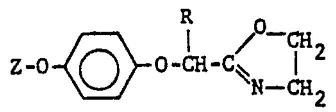

darin bedeuten

R Wasserstoff oder Methyl und

Z einen substituierten Phenylrest- oder Pyrid-2-yl-rest

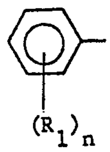
oder
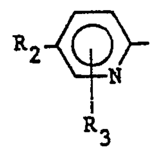

$R_1$ Halogen, Trifluormethyl, Cyano, Nitro,

$R_2$ Wasserstoff, Halogen, Cyano,

$R_3$ Halogen, Trifluormethyl, Cyano und

n die Zahl 1 oder 2.

Unter Halogen wird jedes Halogenatom verstanden, bevorzugt sind Chlor oder Brom.

Aus der US Patentschrift No. 3 877 921 ist bekannt, dass man Phenoxyalkyloxazoline als Herbizide und Wuchsregulatoren verwenden kann. In der DOS 2 613 697 werden herbizide Mittel beschrieben, welche als Wirkstoffe ähnliche Phenoxy-phenoxy-alkyl-heterocyclen enthalten.

Diese Erfindung erschliesst eine neue Gruppe von Phenoxy-alkyl-oxazolidinen, die in geringen Aufwandmengen herbizide Wirkung zeigen.

Die neuen Wirkstoffe der Formel I gemäss vorliegender Erfindung besitzen eine gute, hauptsächlich gegen Gräser gerichtete Herbizidwirkung, vor allem bei post-emergenter Anwendung und können in mono- und dikotylen Kulturen als Unkrautmittel eingesetzt werden.

Die Phenoxy-alkyl-oxazoline vorliegender Erfindung sind wenig giftig für Warmblüter und ihre Anwendung dürfte keine Probleme verursachen. Die vorgeschlagenen Aufwandmengen liegen zwischen 0,1 und 5 kg pro Hektar.

Die Herstellung der neuen Verbindungen der Formel I erfolgt nach an sich bekannten Reaktionen der chemischen Synthese.

Gemäss einem ersten Verfahren werden Phenoxy-alkyl-äthylenimide der Formel II

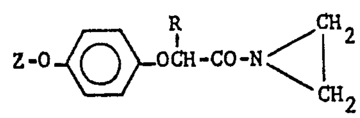
(II)

worin R und Z die unter Formel I gegebene Bedeutung haben, durch Umlagerung des Aziridinyl-Amides unter Einfluss des Jodid-ions in einem Lösungsmittel hergestellt.

Anstelle des Aethylenimides kann auch ein Phenoxy-alkyl-äthylamid der Formel III

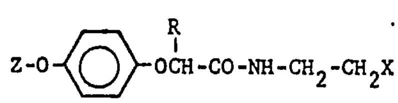
(III)

worin R und Z die unter Formel I gegebene Bedeutung haben, während X die OH-Gruppe, ein Halogenatom oder ein Sulfonsäureesterrest sein kann, in einem Lösungsmittel einem Ringschluss unterworfen werden.

Als Lösungsmittel kommen vor allem aprotische wasserlösliche in Frage, wie niedermolekulare Alkohole, Ketone, Dimethylformamid, Dimethylsulfoxyd aber auch chlorierte Kohlenwasserstoffe.

Der Ringschluss erfolgt in Gegenwart einer Base, wie z.B. einem Alkalymetallhydroxyd oder einer quaternären Ammoniumhydroxyd, wenn X ein Halogenatom oder einen Sulfonsäurerest bedeutet,

jedoch unter sauren Bedingungen, z.B. in Gegenwart von Schwefelsäure, wenn X die Hydroxylgruppe bedeutet.

Als Katalysatoren für den Ringschluss kommen die Alkalimetallsalze oder tertiären Ammoniumsalze von Halogeniden und Sulfonsäuren in Frage.

Der Ringschluss erfolgt bereits bei Raumtemperatur, zur Beschleunigung des Vorganges kann das Reaktionsgemisch bis zum Sieden erwärmt werden.

Ein weiterer Weg, die Phenoxy-alkyl-oxazoline der Formel I herzustellen, besteht darin, dass man einen para-Phenoxy-phenol oder para - Pyrid - 2 - yloxy - phenol der Formel IV

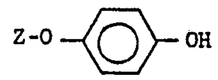

(IV)

worin Z einen unter Formel I definierter Phenyl- oder Pyridylrest bedeutet, mit einem 2-Oxazolin-alkylhalogenid der Formel V

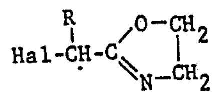

(V)

worin Hal ein Halogenatom bedeutet und

R die unter Formel I gegebene Bedeutung hat, in Gegenwart eines säurebindenden Mittels kondensiert.

Diese Reaktion wird ebenfalls in einem mit Wasser mischbaren Lösungsmittel oder aber in einem Halogenkohlenwasserstoff vorgenommen, bei Normaldruck und einer Temperatur die zwischen Raumtemperatur und dem Siedepunkt des Reaktionsgemisches liegen kann. Vorzugsweise wird dabei unter Ruckfluss gekocht.

Als säurebindendes Mittel können wässerige Alkalimetallhydroxyde wie KOH und NaOH, sowie andere basische Stoffe wie Ammoniak, Karbonate, ($K_2CO_3$, $NaHCO_3$) Alkoholate ($NaOCH_3$, K-tert.butylat) aber auch organische Basen wie Triäthylamin etc. verwendet werden. Falls als Lösungsmittel bereits eine organische Base z.B. Pyridin verwendet wird, son dient dieses zugleich als säurebindendes Mittel.

Die Ausgangsstoffe der Formel II sind am besten durch Amidierung aus den entsprechenden Phenoxy-phenoxy-propionsäuren und Pyridyloxy-phenoxy-propionsäuren, wie sie z.B. in den Deutschen Offenlegungsschriften No. 2 223 894, 2 531 643, 2 546 251 und 2 652 384 beschrieben worden sind.

Die Amidierung geschieht nach an sich bekannten Methoden durch Umsetzen der Halogenide oder des Anhydrides dieser Säuren mit Aethylenimin, den entsprechenden Halogenäthylaminen oder einem 2-Amino-äthyl-sulfonat.

Die nachfolgenden Beispiele veranschaulichen ein erfindungsgemässes Herstellungsverfahren für solche Verbindungen. Die Temperaturen darin sind in Celsiusgraden angegeben, Teile und Prozentangaben beziehen sich auf das Gewicht. Weitere in entsprechender Weise hergestellte Verbindungen sind in der anschliessenden Tabelle aufgeführt.

## Beispiel 1

2-{1-[4'-(3'',5''-dichlor-pyrid-2''-yloxy)-phenoxy]äthyl}-oxazolin

Zu 39 g (0,1 Mol) $\alpha$ - [4 - (3',5' - Dichlor - pyrid - 2'yl) - oxy - phenoxy] - propionsäure - chloräthylamid in 500 ml Methylenchlorid werden 50 g 50%ige NaOH und 1 g Tetrabutylammoniumbromid zugegeben. Nach 4-stündigem Rühren bei Raumtemperatur werden 50 ml Wasser zugegeben, die Methylenchloridphase abgetrennt, mit Magnesiumsulfat getrocknet und eingedampft. Zum öligen Rückstand wird wenig Aether zugegeben. Das dabei kristallisierende Produkt wird abfiltriert und getrocknet. Man erhält 25,3 g Endprodukt mit einem Schmelzpunkt von 111—113°.

## Beispiel 2

2-{1-[4'-(4''-Trifluormethylphenoxy)-phenoxy]äthyl}-oxazolin

Eine Suspension von 25,4 g 4 - (4' - Trifluoromethyl - phenoxy) - phenol, 23,6 g $\alpha$ - Brompropionsäure - 2 - chloräthylamid und 20 g wasserfreiem Kaliumcarbonat in 250 ml Aethylenmethylketon wird während 18 Std. bei Rückflusstemperatur gerührt, filtriert und das Filtrat am Vakuum eingedampft. Der ölige Rückstand (19,5 g) wird in 75 ml Methylenchlorid gelöst, mit 2,5 g Tetrabutylammonium-hydrogensulfat und 2,51 g Natriumhydroxyd in 25 ml Wasser versetzt und unter Stickstoffatmosphäre während 3 Std. bei Raumtemperatur gerührt. Die Methylenchloridphase wird abgetrennt, mit Wasser gewaschen, getrocknet und am Vakuum eingedampft. Man erhält 13,6 g der Titelverbindung $n_D^{20}$ 1.5274.

In analoger Weise werden folgende Verbindungen hergestellt:

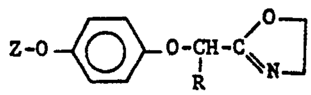

| Verbindung No. | Z | R | Physikal. Daten |
|---|---|---|---|
| 3 | 2-Chlor-4-trifluormethylphenyl | $CH_3$ | $n_D^{20}$: 1,5391 |
| 4 | 2,4-Dichlorphenyl | $CH_3$ | $n_D^{30}$: 1,5764 |
| 5 | 3,5-Dichlor-pyridyl-(2) | H | |
| 6 | 4-Trifluormethylphenyl | H | |
| 7 | 4-Chlor-2-cyano-phenyl | $CH_3$ | $n_D^{23}$: 1,5751 |
| 8 | 4-Chlor-phenyl | $CH_3$ | $n_D^{23}$: 1,5678 |
| 9 | 4-Cyano-phenyl | $CH_3$ | $n_D^{23}$: 1,5732 |
| 10 | 4-Chlor-2-trifluormethylphenyl | | |
| 11 | 4-Chlor-2-nitrophenyl | $CH_3$ | $n_D^{23}$: 1,5852 |
| 12 | 4-Brom-2--itrophenyl | $CH_3$ | |

Die neuen $\alpha$ - [4 - (Phenoxy - phenoxy] - und $\alpha$ - [4 - (Pyridyloxy) - phenoxy] - alkyl - oxazoline zeigen bei relativ niederen Aufwandmengen eine starke herbizide Wirkung.

Die neuen Wirkstoffe der Formel I sind als Herbizide im Vorauflauf-, als insbesondere auch im Nachauflauf- Verfahren brauchbar. Ihre Wirkung richtet sich vor allem gegen monocotyle Unkräuter, sie können zur selektiven Unkrautbekämpfung bei dikotylen Pflanzenkulturen eingesetzt werden, wie z.B. in Soja. Sie eignen sich jedoch ebenfalls zur selektiven Unkrautbekämpfung in gewissen monocotyledonen Kulturen, z.B. in Getreiden wie Weizen und Gerste.

Die Verwendungen sind wenig giftig für Warmblüter; die Aufwandmengen liegen vorteilhaft zwischen 0,1 und 5 kg Wirkstoff pro Hektar.

Die vorliegende Erfindung betrifft auch herbizide Mittel, welche eine Verbindung der Formel I als aktive Komponente enthalten. Solche Mittel können in üblicher Weise als feste Formulierungen (Stäubemittel, Streumittel, Granulate), als in Wasser dispergierbare Konzentrate (Spritzpulver, Emulsionen, Emulsionskonzentrate und Pasten) oder als Lösungen vorliegen und werden nach bekannten Techniken mit entsprechenden Zuschlag- und Trägerstoffen formuliert.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenuber den Wirkstoffen inerten Dispersions- oder Lösungsmitteln. Die Wirkstoffe können in den folgenden Aufarbeitungsformen vorliegen und angewendet werden:

feste Aufarbeitungsformen:
Stäubemittel, Streumittel, Granulate, Umhüllungsgranulate Imprägnierungsgranulate und Homogengranulate;

in Wasser dispergierbare Wirkstoffkonzentrate:
Spritzpulver, (wettable powder), Pasten, Emulsionen:

flüssige Aufarbeitungsformen:
Lösungen.

Der Gehalt an Wirkstoff in den oben beschriebenen Mitteln liegt zwischen 0,1 bis 95%, bevorzugt zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Die Wirkstoffe der Formel I können beispielsweise wie folgt formuliert werden (Teile bedeuten Gewichtsteile):

*Stäubemittel:*

Zur Herstellung eines a) 5%igen und b) 2%igen Stäubemittels werden die folgenden Stoffe verwendet:

| | | | |
|---|---|---|---|
| | 5 | Teile | eines der Wirkstoffe der Formel I |
| | 95 | Teile | Talkum |
| b) | 2 | Teile | des obigen Wirkstoffes |
| | 1 | Teil | hochdisperse Kieselsäure, |
| | 97 | Teile | Talkum; |

Die Wirkstoffe werden mit den Trägerstoffen vermischt und vermahlen.

*Granulate*

Zur Herstellung eines 5%igen Granulates werden die folgenden Stoffe verwendet:

| | | |
|---|---|---|
| 5 | Teile | eines der Wirkstoffe der Formel I |
| 0,25 | Teile | Epichlorhydrin |
| 0,25 | Teile | Cetylpoläthylenglykoläther mit 8 Mol Aethylenoxid, |
| 3,50 | Teile | Polyäthylenglykol, |
| 91 | Teile | Kaolin (Korngrösse 0,3 bis 0,8 mm) |

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

*Spritzpulver*

Zur Herstellung eines a) 50%igen, b) 25%igen und c) 10%igen Spritzpulvers werden folgende Bestandteile verwendet:

| | | | |
|---|---|---|---|
| a) | 50 | Teile | eines der Wirkstoffe der Formel I |
| | 5 | Teile | Natriumdibutylnaphthylsulfonat, |
| | 3 | Teile | Naphthalinsulfonsäuren-Phenonolsulfonsäuren-Formaldehyd-Kondensat 3:2:1, |
| | 20 | Teile | Kaolin, |
| | 22 | Teile | Champagne-Kreide; |
| b) | 25 | Teile | des obigen Wirkstoffes, |
| | 5 | Teile | Oleylmethyltaurid-Natrium-Salz, |
| | 2,5 | Teile | Naphthalinsulfonsäuren-Formaldehyd-Kondensat, |
| | 0,5 | Teile | Carboxymethylcellulose, |
| | 5 | Teile | neutrales Kalium-Aluminium-Silikat, |
| | 62 | Teile | Kaolin; |
| c) | 10 | Teile | des obigen Wirkstoffs, |
| | 3 | Teile | Gemisch der Natriumsalze von gesättigten Fettalkoholen, |

5 Teile Naphthalinsulfonsäuren-Formaldehyd-Kondensat,

82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen im Vorauflaufverfahren verwendet.

*Paste*

Zur Herstellung einer 45%igen Paste werden folgende Stoffe verwendet:

45 Teile eines der Wirkstoffe der Formel I

5 Teile Natriumaluminiumsilikat,

14 Teile Cetylpolyäthylenglykoläther mit 8 Mol Aethylenoxid,

1 Teil Oleylpolyäthylenglykoläther mit 5 Mol Aethylenoxid,

2 Teile Spindelöl,

23 Teile Wasser,

10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen. Die Suspension eignen sich zur Behandlung von Rasenanlagen.

*Emulsionskonzentrat*

Zur Herstellung eines 25%igen Emulsionskonzentrates werden

25 Teile eines der Wirkstoffe der Formel I

5 Teile Mischung von Nonylphenolpolyoxyäthylen und Calcium-dodecylbenzol-sulfonat,

35 Teile 3,5,5-Trimethyl-2-cyclohexen-1-on,

35 Teile Dimethylformamid

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere der von der Formel I umfassten Verbindungen verwendet werden.

Die erfindungsgemässen Wirkstoffe sind auch interessante Kombinationspartner für eine Reihe von Herbiziden der Phenylharnstoff- und Triazinreihe in Getreidekulturen, Mais, Zuckerrohr bzw. im Obst- und Weinbau.

Zum Nachweis der herbiziden Wirkung wurde im Gewächshaus die Wirkung der Verbindung

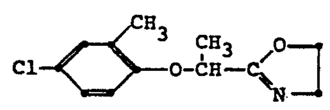

A

2 - [1' - (4'' - Chlor - 2'' - methylphenoxy) - äthyl] - 2 - oxazolin bekannt aus dem US Patent No. 3,778,445, Beispiel 9 mit derjenigen der erfindungsgemässen Verbindungen No. 1, 2, 3 und 7 verglichen. Die Verbindungen wurden zu 25%-igen Emulsionskonzentraten aufgearbeitet, welche dann vor dem Spritzen mit Wasser zu der gewünschten Wirkstoffkonzentration verdünnt wurden.

Versuchsmethode:

*Post-emergente Herbizid-Wirkung (Kontaktherbizid)*

Die grasartigen Unkräuter (Avena fatua, Rottboellia exaltata und Echinochloa crus galli) wurden im Gewächshaus in Blumentöpfe gesät. Nach dem Auflaufen (wenn sie das 4- bis 6-Blattstadium erreicht haben) wurden sie mit einer wässrigen Wirkstoffemulsion in Dosierungen von 1 und 2 kg Wirkstoff pro Hektar gespritzt. Die Töpfe wurden dann bei 25—26°C und 45—60% relativer Luftfeuchtigkeit gehalten. 15 Tage nach der Behandlung wurde der Versuch ausgewertet und die Resultate nach folgender Notenskala bonitiert:

10 = Pflanzen total abgestorben

7—9 = sehr starke herbizide Wirkung

5 = mittlere Wirkung

1—3 = geringe Wirkung

0 = keine Wirkung (Die Unkräuter gedeihen wie unbehandelte Kontrollpflanzen)

Die Resultate sind in der untenstehenden Tabelle zusammengefasst:

| Pflanze | Avena fatua | | Rottboellia exaltata | | Echinochloa crus galli | |
|---|---|---|---|---|---|---|
| Aufwandmenge in kg/Hektar | 2 | 1 | 2 | 1 | 2 | 1 |
| Wirksubstanz | | | | | | |
| A bekannt | 0 | 0 | 3 | 2 | 5 | 2 |
| 1 | 10 | 10 | 10 | 10 | 10 | 10 |
| 2 | 10 | 10 | 10 | 10 | 10 | 10 |
| 3 | 5 | 3 | 7 | 6 | 10 | 10 |
| 7 | 5 | 4 | 4 | 2 | 10 | 10 |

In diesem Versuch zeigen die erfindungsgemässen Verbindungen 1 und 2 starke Herbizidwirkung gegen alle 3, die Verbindung 3 gegen 2 und die Verbindzug 7 gegen eines der geprüften Unkräuter, während die vorbekannte Verbindung A bloss gegen Echinochloa crus galli bei einer Aufwandmenge von 2 kg pro Hektar einigermassen wirkt.

*Post-emergente Wirkung im Feld*

Ein im Oktober mit Winterweizen der Sorte "TAM 101" angesätes Feld wurde im nächsten Frühjahr, im März, (5 Monate nach der Saat), wenn der Weizen das 2—3 Blatt-Stadium erreicht hatte und avena fatua (Flughafer) das prominenteste Unkraut im 1—5 Blatt-Stadium anzutreffen war, parzellenweise mit verdünnten wässrigen Wirkstoffemulsionen behandelt, so dass die Parzellen je mit 2, 1.5, 1, 0.75 und 0.5 kg Wirkstoff pro Hektar besprüht wurden. Der Versuch wurde nach 37 Tagen, Ende Arpil ausgewertet und der Zustand des Weizens und des Flughafers gemäss der obenstehenden Skala bonitiert. Die Resultate sind in der unterstehenden Tabelle zusammengefasst:

| Aufwandmenge kg/ha | Winterweizen 2 | 1.5 | 1 | 0.75 | 0.5 | avena fatua 2 | 1.5 | 1 | 0.75 | 0.5 |
|---|---|---|---|---|---|---|---|---|---|---|
| Wirksubstanz | | | | | | | | | | |
| No. 1 | 5 | 3 | 2 | 0 | 0 | 10 | 10 | 9 | 9 | 9 |
| B | 8 | 7 | 7 | 7 | 5 | 10 | 10 | 10 | 10 | 10 |
| C | 3 | 2 | 2 | – | 2 | 8 | 8 | 7 | – | 3 |

B = 2-[5-(3',5'-Dichlorpyridyl-2'-oxy)-phenoxy)]-propionsaure-methylester bekannt aus der DOS 2 546 251 oder der USP 4 046 553.

C = 2-[4-(2',4'-Dichlorphenoxy)-phenoxy]-propionsaure-methylester (HOE 23 408), bekannt aus DOS 2 223 894 oder USP 3 953 442.

– = Diese Konzentration wurde nicht geprüft.

Eine Mitte Februar mit Sommergeste vom Typ "CM-67" angesätes Feld wurde nach 42 Tagen, Ende März, als die Gerste das 4—6 Blatt-Stadium und avena fatua, das am meisten verbreitete Unkraut das 5—7 Blatt-Stadium erreicht hatten, parzellenweise mit verdünnten wässerigen Wirkstoffemulsionen behandelt, so dass die Parzellen je mit 2, 1.5, 1, 0.75 und 5 kg Wirkstoff pro Hektar besprüht wurden. Der Versuch wurde nach 22 Tagen, im April ausgewertet und der Zustand der Pflanzen gemäss der oben gegebenen Skala bonitiert. Die Resultate sind in der untenstehenden Tabelle zusammengefasst.

| Aufwandmenge in kg/ha | Sommergerste 2 | 1.5 | 1 | 0.75 | 0.5 | avena fatua 2 | 1.5 | 1 | 0.75 | 0.5 |
|---|---|---|---|---|---|---|---|---|---|---|
| Wirksubstanz | | | | | | | | | | |
| No. 1 | 0 | 0 | 0 | 0 | 0 | 10 | 10 | 10 | 10 | 10 |
| B | 9 | 9 | 9 | 7 | 6 | 10 | 10 | 10 | 10 | 10 |
| C | 0 | 0 | 0 | – | 0 | 10 | 9 | 6 | – | 5 |

– = Diese Konzentration wurde nicht geprüft.

Die Verbindung No. 1 vermag avena fatua zu vernichten ohne die Sommergerste oder bei Aufwandmengen von 1 kg/ha und weniger, den Winterweizen zu schädigen.

**Patentansprüche**

1. Phenoxy-alkyl-oxazoline der Formel I

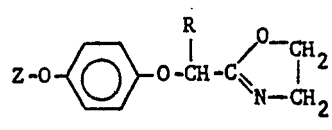

worin
R Wasserstoff oder Methyl und
Z einen substituierten Phenyl- oder Pyrid-2-ylrest,

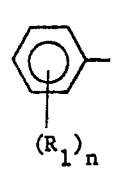
oder
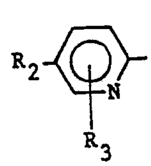

$R_1$ Halogen, Trifluormethyl, Cyano, Nitro,
$R_2$ Wasserstoff, Halogen, Cyano,

$R_3$ Halogen, Trifluormethyl, Cyano,
n die Zahl 1 oder 2 bedeuten.

2. 2-{1-[4'-(3'',5''-Dichlorpyrid-2''-yloxy)-phenoxy]-äthyl}-oxazolin, gemäss Anspruch 1.

3. 2-{1-[4'-(4''-Trifluormethylphenoxy)-phenoxy]-äthyl}-oxazolin, gemäss Anspruch 1.

4. Verfahren zur Herstellung der Phenoxy-alkyl-oxazolinen der Formel I, Anspruch 1, dadurch gekennzeichnet, dass man in einem Phenoxy-alkyl-äthylenimid der Formel II

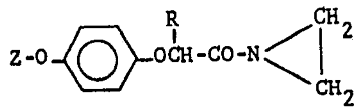

(II)

worin R und Z die unter Formel I, Anspruch 1 gegebene Bedeutung haben, den Aziridinylring in einem Lösungsmittel unter Einfluss des Jodid-ions zum Oxazolin-Ring umlagert.

5. Verfahren zur Herstellung der Phenoxy-alkyl-oxazoline der Formel I, Anspruch 1, dadurch gekennzeichnet, dass man ein Phenoxy-alkyl-äthylamid der Formel III

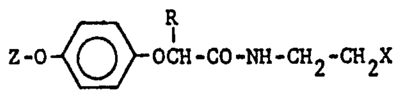

(III)

worin R und Z die unter Formel I, Anspruch 1 gegebene Bedeutung haben, während X die OH-Gruppe, ein Halogenatom oder einen Sulfonsäurerest bedeuten kann, in einem Lösungsmittel zum Ringschluss bringt.

6. Verfahren zur Herstellung der Phenoxy-alkyl-oxazoline der Formel I, Anspruch 1, dadurch gekennzeichnet, dass man ein para-Phenoxy-phenol oder ein para-Pyrid-2-yloxy-phenol der Formel IV

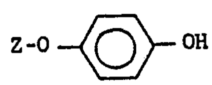

(IV)

worin Z die unter Formel I, Anspruch 1 gegebene Bedeutung hat, mit einem 2-Oxazolin-alkyl-halogenid der Formel V

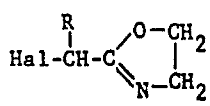

(V)

worin

Hal ein Halogenatom bedeutet und

R die unter Formel I, Anspruch 1 gegebene Bedeutung hat, in Gegenwart eines säurebindenden Mittels kondensiert.

7. Herbizides Mittel, dadurch gekennzeichnet, dass es als aktive Komponente ein Phenoxy-alkyloxazolidin der Formel I, Anspruch 1 enthält.

8. Die Verwendung der Phenoxy-alkan-oxazolidine der Formel I, Anspruch 1 oder sie enthaltender Mittel zur Bekämpfung von Unkräutern in Kulturpflanzungen.

9. Die Verwendung der Phenoxy-alkan-oxazoline der Formel I, Anspruch 1 oder sie enthaltender Mittel zur Bekämpfung von Unkräutern in Kulturen von Getreiden und Soja.

**Revendications**

1. Phénoxy-alcoyl-oxazolines de formule I

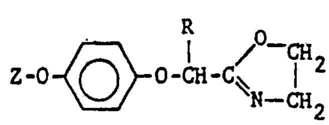

où

R représente un hydrogène ou un méthyle et
Z un radical phényle ou pyrid-2-yle substitué

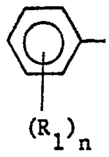

ou

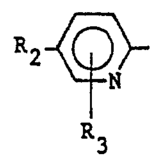

$R_1$ un halogène, un trifluorométhyle, un cyano ou un nitro,
$R_2$ un hydrogène, un halogène ou un cyano,
$R_3$ un halogène, un trifluorométhyle ou un cyano et n le nombre 1 ou 2.

2. 2-[1-[4'-(3'',5''-dichloropyrid-2''-yloxy)-phénoxy]-éthyl]-oxazoline selon la revendication 1.

3. 2-[1-[4'-(4''-trifluorométhylphénoxy)-phénoxy]-éthyl]-oxazoline selon la revendication 1.

4. Procédé de préparation des phénoxy-alcoyl-oxazolines de formule I de la revendication 1, caractérisé en ce que dans un phénoxy-alcoyl-éthylénimide de formule II

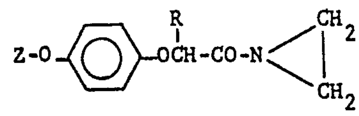

(II)

où R et Z ont la signification donnée pour la formule I de la revendication 1, on transpose le noyau aziridinyle dans un solvant sous l'influence de l'ion iodure au noyau oxazoline.

5. Procédé de préparation des phénoxy-alcoyl-oxazolines de formule I de la revendication 1, caractérisé en ce qu'on cyclise un phénoxy-alcoyl-éthylamide de formule III

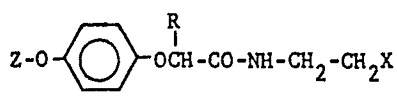

(III)

où R et Z ont la signification donnée pour la formule I de la revendication 1, cependant que X peut représenter le groupe OH, un atome d'halogène ou un radical acide sulfonique, dans un solvant.

6. Procédé de préparation des phénoxy-alcoyl-oxazolines de formule I de la revendication 1, caractérisé en ce qu'on condense un para-phénoxy-phénol ou un para - pyrid - 2 - yloxy - phénol de formule IV

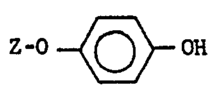

(IV)

où Z a la signification donnée sous la formule I de la revendication 1, avec un halogénure de 2-oxazolin-alcoyle de formule V

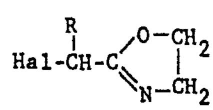

(V)

où Hal représente un atome d'halogène et où R a la signification donnée pour la formule I de la revendication 1, en présence d'un agent liant acide.

7. Agent herbicide, caractérisé en ce qu'il contient comme composant actif une phénoxy-alcoyl-oxazolidine de formule I de la revendication 1.

8. Application des phénoxy-alcoyl-oxazolidines de formule I de la revendication 1 ou des agents qui les contiennent à la lutte contre les mauvaises herbes dans les plantes cultivées.

9. Application des phénoxy-alcoyl-oxazolines de formule I de la revendication 1 ou des agents qui les contiennent à la lutte contre les mauvaises herbes dans les cultures de céréales et de soja.

**Claims**

1. A phenoxyalkyloxazoline of the formula I

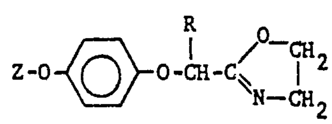

wherein

R is hydrogen or methyl and
Z is the substituted phenyl radical or pyrid-2-yl radical

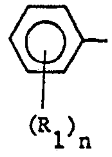 or 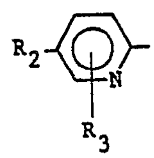

wherein
$R_1$ is halogen, trifluoromethyl, cyano or nitro,
$R_2$ is hydrogen, halogen or cyano,
$R_3$ is halogen, trifluoromethyl or cyano, and
n is 1 or 2.

2. 2-{1-[4′-(3″,5″-Dichloropyrid-2″-yloxy)-phenoxy]-ethyl}-oxazoline according to claim 1.

3. 2-{1-[4′-(4″-Trifluoromethylphenoxy)-phenoxy]-ethyl}-oxazoline according to claim 1.

4. A process for the production of a phenoxyalkyloxazoline of the formula I according to claim 1, which process comprises rearranging the aziridinyl ring in a phenoxyalkylethyleneimide of the formula II

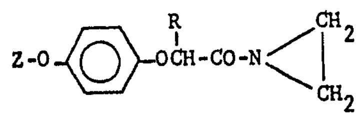

(II)

wherein R and Z are as defined for formula I in claim 1, in a solvent, under the influence of the iodide ion, to form the oxazoline ring.

5. A process for the production of a phenoxyalkyloxazoline of the formula I according to claim 1, which process comprises cyclising a phenoxyalkylethylamide of the formula III

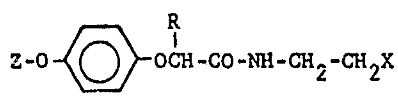

(III)

wherein R and Z are as defined for formula I in claim 1, whilst X can be the OH group, a halogen atom or a sulfonic acid radical, in a solvent.

6. A process for the production of a phenoxyalkyloxazoline of the formula I according to claim 1, which process comprises condensing a para-phenoxyphenol or a para -(pyrid -2 -yloxy) -phenol of the formula IV

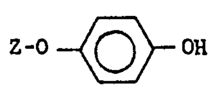

(IV)

wherein Z is as defined for formula I in claim 1, with a 2-oxazoline-alkyl halide of the formula V

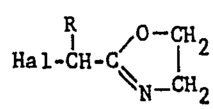

(V)

wherein Hal is a halogen atom and R is as defined for formula I in claim 1, in the presence of an acid acceptor.

7. A herbicidal composition which contains, as active component, a phenoxyalkyloxazoline of the formula I according to claim 1.

8. A method of controlling weeds in crops of cultivated plants, which comprises applying thereto a phenoxyalkyloxazoline of the formula I according to claim 1 or a composition containing such a compound.

9. A method of controlling weeds in crops of cereals and soya, which comprises applying thereto a phenoxyalkyloxazoline of the formula I according to claim 1 or a composition containing such a compound.